(19) **Europäisches Patentamt**
**European Patent Office**
**Office européen des brevets**

(11) **EP 4 624 971 A1**

(12) **EUROPEAN PATENT APPLICATION**

(43) Date of publication:
**01.10.2025 Bulletin 2025/40**

(21) Application number: **24167445.6**

(22) Date of filing: **28.03.2024**

(51) International Patent Classification (IPC):
**G01R 33/54** (2006.01)     **G01R 33/56** (2006.01)
**G01R 33/567** (2006.01)

(52) Cooperative Patent Classification (CPC):
**G01R 33/5608; G01R 33/54; G01R 33/5676;**
G01R 33/543; G01R 33/546

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Siemens Healthineers AG**
  **91301 Forchheim (DE)**
• **Friedrich-Alexander-Universität Erlangen-
  Nürnberg
  91054 Erlangen (DE)**

(72) Inventors:
• **Cauley, Stephen Farman**
  **Winchester, 01890 (US)**
• **Clifford, Bryan**
  **Belmont, 02478 (US)**
• **Hossbach, Julian**
  **91058 Erlangen (DE)**
• **Lo, Wei-Ching**
  **Charlestown, 02129 (US)**
• **Polak, Daniel**
  **91052 Erlangen (DE)**
• **Splitthoff, Daniel Nicolas**
  **91080 Uttenreuth (DE)**

(74) Representative: **Siemens Healthineers
Patent Attorneys
Postfach 22 16 34
80506 München (DE)**

(54) **METHOD FOR ACQUIRING A MAGNETIC RESONANCE IMAGE DATASET OF A BODY PART OF A SUBJECT**

(57)     The invention relates to a method for acquiring a magnetic resonance image dataset of a body part of a subject (U), the method comprising the steps of: (a) acquiring a low-resolution magnetic resonance image (40) of the body part, (b1) optionally acquiring a reference set (38) of additional k-space lines within a central region (16) of k-space, (b2) acquiring further sets (36) of additional k-space lines (12) within a central region (16) of k-space at intervals throughout the imaging protocol, (c) applying a trained machine learning model (42) to a dataset (34) comprising the low-resolution image (40) in k-space notation and a further set of additional k-space lines (36), wherein a set of pose parameters (44) is generated, and (d) using the pose parameters (44) for prospective (46) and/or retrospective motion correction of the magnetic resonance image dataset.

FIG 3

**Description**

Independent of the grammatical term usage, individuals with male, female or other gender identities are included within the term.

The invention relates to a method for acquiring a magnetic resonance image dataset of a body part of a subject, a computer-implemented method for providing a trained machine learning model, a computer program and a magnetic resonance imaging apparatus.

Patient motion is one of the most common and costly type of artefacts in Magnetic Resonance Imaging (MRI) and can seriously degrade the diagnostic quality of magnetic resonance (MR) examinations.

Motion correction can be done retrospectively, i.e. after the acquisition of the magnetic resonance image dataset, or prospectively, i.e. during the acquisition. Both approaches try to minimize the effect of motion and mitigate motion artifacts, but often lack the applicability for the clinical routine.

Retrospective methods correct for motion artefacts after the data acquisition in the course of the image reconstruction. By including motion parameters into the MR forward model, these techniques account for the patient's motion in the final image reconstruction and therefore reduce motion artefacts. In some techniques, the motion data can be derived from the acquired k-space data itself. For multi-shot acquisitions, the goal in retrospective motion correction techniques is to extract the *per shot* motion parameters and the motion-free image simultaneously. This can be accomplished by either minimizing an image quality metric, such as image entropy, or by minimizing the data consistency error of a so-called "SENSE + motion" forward model, as described e.g. in L. Cordero-Grande, R. Teixeira, E. Hughes, J. Hutter, A. Price, Hajnal, "Sensitivity Encoding for Aligned Multishot Magnetic Resonance Reconstruction," IEEE Trans. Comput. Imaging, vol. 2, no. 3, pp. 266-280, 2016.

In "Scout accelerated motion estimation and reduction (SAMER)", Magn. Reson. Med., vol. 87, pp. 163-178, 2022, https://doi.org/10.1002/mrm.28971, D. Polak, D. N. Splitthoff, B. Clifford, W.-C. Lo, S. Huang, J. Conklin, L. L. Wald, K. Setsompop and S. Cauley propose a technique that utilizes a single rapid scout scan to drastically reduce the computational cost of motion estimation. The scout image contains centre of k-space information which is compared against the k-space data of the actual MR acquisition for each shot, to derive the subject's motion. This strategy is used to completely avoid the alternating optimization of subject motion parameters and image volume, which is otherwise required in other data-consistency based retrospective motion correction techniques. In the SAMER-technique, a motion trajectory of the subject is first estimated, and the motion trajectory is then used in a motion-aware parallel image reconstruction, using e.g. a "SENSE + motion" forward model, to yield the motion-mitigated image. In D. Polak, J. Hossbach, D. N. Splitthoff et al. "Motion guidance lines for robust data consistency-based retrospective motion correction in 2D and 3D MRI", Magn. Reson. Med. 2023:1-14, doi: 10.1002/mrm.29534, D. Polak et al. have extended the SAMER method to include the repeated acquisition of a small number of motion guidance lines in each shot, which are used for motion estimation by being compared with the data from the scout scan. This allows an estimation of motion parameters shot-by-shot.

Retrospective motion correction techniques often require a significant processing time and thereby may increase the reconstruction time to a level that is unacceptable in clinical routine. Furthermore, rotation and spin-history artifacts are not always fully correctable retrospectively.

Prospective motion correction requires an accurate tracking of the subject's motion. This can be done by additional hardware to track patient motion during the MR examination, e.g., cameras, or separate navigator scans. Camera-based approaches might lack the accuracy to differentiate skin motion from rigid body motion, in particular of the head. Navigators require thorough sequence changes potentially prolonging the acquisition, as explained in Maclaren, J., Herbst, M., Speck, O. et al. Prospective motion correction in brain imaging: A review. Magn Reson Med, 2013; 69:621-636.

Recently, deep learning techniques have increasingly been applied to image reconstruction in MRI. In Hossbach J, Splitthoff D, Cauley S, et al. "Deep Learning-Based Motion Quantification from k-space for Fast Model-Based MRI Motion Correction". Med. Phys., 2023; 50:2148-2161, it has been demonstrated that it is possible to use a neural network to estimate subject motion between two acquisitions. However, the method requires the use of special sequence features, such as echo trains that span over the full extent of k-space; i.e. each echo train has data in the low-resolution part of k-space. The method cannot be universally applied to any imaging sequence.

It is therefore an object of the invention to alleviate the above-mentioned problems of prior art motion techniques. It is in particular an object of the invention to provide a method for acquiring a motion corrected MRI dataset of a body part of a subject, in which motion correction does not delay the acquisition or reconstruction of the image data, which works with any type of imaging sequence, and which provides a good motion correction performance.

These objects are met or exceeded by a method for acquiring a motion-corrected MRI dataset according to claim 1, a computer implemented method for providing a trained machine learning model according to claim 13, a computer program and an MRI apparatus according to claim 15.

According to a first aspect, the invention is directed to a method for acquiring a magnetic resonance image dataset of a body part of a subject, wherein the magnetic resonance image dataset is acquired using an imaging protocol

wherein spatial encoding is performed using phase encoding gradients along at least one phase encoding direction, and frequency encoding gradients along a frequency encoding direction, and wherein k-space is sampled during the imaging protocol by acquiring a plurality of imaging k-space lines oriented along the frequency encoding direction, and having different positions in the at least one phase encoding direction, the method comprising the steps of:

(a) acquiring or providing a low-resolution magnetic resonance image of the body part,
(b1) optionally acquiring a reference set of additional k-space lines within a central region of k-space, preferably at the beginning of the imaging protocol,
(b2) acquiring further sets of additional k-space lines within a central region of k-space at intervals throughout the imaging protocol,
(c) applying a trained machine learning model to a dataset comprising the low-resolution image in k-space notation and a further set of additional k-space lines, wherein a set of pose parameters is generated, the pose parameters being an estimation of the pose of the subject during the acquisition time of the further set of additional k-space lines.

The invention uses a trained machine learning model, for example a neural network (NN), to estimate pose parameters from the additional k-space lines, which are acquired in addition to the imaging protocol, in a similar fashion as in the SAMER technique. The set of additional k-space lines is acquired at intervals throughout the imaging protocol, and those additional k-space lines - also referred to as guidance lines (GL) - are used to estimate pose parameters, referred to herein as motion parameters. The estimated pose parameters provide a sufficiently good estimate of the varying position of the imaged body part throughout the imaging protocol. A time sequence of pose parameters may also be referred to as motion trajectory.

In the above-described SAMER technique, such a motion trajectory is used in the image reconstruction in order to allow retrospective motion correction. However, the SAMER technique has the disadvantage that the pose parameters are estimated by the registration of the additionally acquired guidance lines to a previously acquired scout image. This motion estimation is performed by numerically solving an optimization problem which requires as input processed data such as coil sensitivities and the reconstructed scout, leading to a processing time which is usually too long to instantly allow use of the estimated pose parameters for prospective motion correction, i.e. right from the start of the sequence while the imaging protocol is being performed. Rather, the SAMER technique is usually limited to retrospective motion correction. The invention provides a significant improvement over the SAMER method by applying a trained machine learning model to a dataset comprising the guidance lines and a low-resolution image of the body part, wherein the output of the trained machine learning model is a set of pose parameters. This step in k-space is significantly faster than the previously used numerical optimization, in which each set of pose parameters was determined independently by minimizing the data consistently error of a forward model, for example a SENSE + motion forward model, which is computationally more demanding, since it involves both image and k-space operations. The trained machine learning model advantageously operates in k-space, so that both the low-resolution image and the guidance lines are inputted in k-space notation. This has the additional advantage that no scout image reconstruction is required and the preprocessing of the dataset, to which the trained machine learning model is applied, can be kept to a minimum. For example, no coil sensitivity maps calculation is required. This further reduces the processing time required for the estimation of the pose parameters. Overall, the time required for estimating pose parameters from a set of additional k-space lines (guidance lines) is so small, preferably less than 15ms, more preferred less than 8ms, that the pose parameters can be used for prospective motion correction of the magnetic resonance image dataset. In particular, the pose parameters can be used to adapt a field of view of the MRI dataset during the imaging protocol. In other words, if the patient has moved during the acquisition of an MRI image dataset, this is reflected in the next set of pose parameters, and the acquisition parameters can be immediately adapted to the new position for the rest of the acquisition. This prospective motion correction provides better results than retrospective motion correction, because it avoids gaps in k-space in the acquired data, which may otherwise occur, and which are not fully correctable retrospectively.

In addition, it has been found that the use of a trained machine learning model for the estimation of posed parameters is advantageous over the previously used numerical optimization. In particular, it has proven to work even if the low-resolution image and the additional k-space lines, to which the trained machine learning model is applied, have different contrasts. Different contrasts may arise if the low-resolution image and the guidance lines have been acquired for example at different echo times and/or using different magnetization preparation and/or different repetition times, in other words, different sequence parameters or a different type of imaging sequence. In the SAMER technique, the scout image used in the estimation of the motion trajectory is specifically acquired for this purpose and will therefore have the same contrast as the motion guidance lines acquired throughout the imaging protocol. This necessarily requires a dedicated scout image and thus additional scan time. According to the invention, however, any low-resolution image of the body part can be used for training the machine learning model. Thus, instead of being limited to a scout reference image, as in the conventional SAMER technique, any other fully sampled prior scan or calibration measurement can be used by the trained machine learning model. The obtained motion parameters can be used to adapt the field of view during the acquisition of MRI dataset for prospective motion correction. Optionally, the guidance lines can be continued to be acquired in the original field of view without any adaptation. The pose parameters can in addition or alternatively be used in retrospective motion correction. In particular, the method of the invention is seamlessly compatible with an additional retrospective SAMER correction. Thus, an additional retrospective or even a purely retrospective application of the proposed trained machine learning model is possible.

The invention uses a fast trained machine learning model, in particular neural network, which is applied to a dataset comprising the SAMER guidance line data. Since the invention operates exclusively in k-space, there is no need for a prior reconstruction of data, making the method particularly fast. The obtained pose parameters can be readily used for prospective motion correction. This minimizes motion artifacts and second order artifacts, such as spin history artifacts, as well as k-space gaps, caused for example by rotational patient motion. A normal reconstruction (e.g. GRAPPA) can be used after the prospective correction, providing the motion corrected MR dataset almost immediately.

The inventive method may be executed on any medical or other MRI apparatus. The motion-corrected MR image is acquired from a field-of-view including a body part of a subject. The subject may be human or animal, in particular a patient to be examined. The image dataset is in particular acquired from a part of the body that is subject to undesired motion, for example the head or neck, a limb such as a leg, arm, knee, hand, or a part subjected to breathing motion such as the thorax or abdomen. The image dataset may be a three-dimensional (3D) dataset, acquired using two phase encoding directions, or it may be a two-dimensional (2D) image dataset, wherein a 2D image dataset comprises one or preferably a stack of 2D slices. A 2D image dataset is acquired using a slice selection gradient typically followed be phase encoding in one in-plane direction and frequency encoding in the other in-plane direction.

The imaging protocol may use any type of imaging sequence, for example a spin-echo or gradient echo sequence. It may for example be a TSE or TSE-type sequence, for example having T1-weighted, T2-weighted or other contrast. It may be a non-steady-state sequence, i. e. one in which the signal intensity or contrast varies over an echo train in particular due to T1 and/or T2 relaxation, such as MPRAGE (Magnetization Prepared Rapid Gradient Echo Imaging) or TSE (Turbo Spin-Echo). Further examples of the imaging protocol are a SPACE sequence (Sampling Perfection with Application optimized Contrast using different flip angle Evolution), SWI (Susceptibility-Weighted Imaging) and FLAIR (Fluid-attenuated inversion recovery) sequence, but other types of imaging sequences are also possible, in particular various gradient echo sequences.

The thereby acquired MR image dataset, also referred to as "image dataset", "diagnostic image datset" or "high-resolution image", may be reconstructed using retrospective motion correction techniques, in particular the technique disclosed by D. Polak et al. in Magn. Reson. Med., vol. 87, pp. 163-178, 2022 and Magn. Reson. Med. 2023:1-14, doi: 10.1002/mrm.29534. The image dataset is typically acquired for diagnostic purposes and thus preferably has a high spatial resolution of, for example, an in-plane resolution of 0.3mm - 3mm, preferably 0.4-2mm. The voxel size may be for example e.g. 0.5 to $12mm^3$, preferably 2 to $8mm^3$. For a 2D image, the in-plane resolution may for example be 0.3mm - 2mm, preferably 0.4-1.2mm.

The low-resolution image used in the method also covers the body part. It has an overlapping field-of-view to the MR image dataset, and it may have the same field-of-view, but not necessarily. It is an advantage of the invention that the low-resolution image does not have to be perfectly matched in terms of field-of-view and contrast to the guidance lines and MR image dataset. In case the image dataset is a stack of 2D slices, the low-resolution image may also comprise a stack of low-resolution 2D slices. However, it is possible that the low-resolution image comprises fewer slices than the high-resolution image, for example only every second or third slice. The low-resolution image may have a spatial resolution of 2-8mm, preferably 3-5mm, for example 4mm, in the phase-encoding direction(s). Preferably, the acquisition of the low-resolution image is very rapid, requiring e.g. 1-5 sec, preferably 1-2 secs. The low-resolution image may be acquired in the course of a calibration imaging scan, which is normally always acquired at the beginning of a patient examination, e.g. after the patient as been positioned in the MR scanner, and before the diagnostic scans are performed. For example, the low-resolution image may be an imaging scan acquired to estimate the sensitivity profile of a multi-channel local coil which is used in that particular examination (PatRefScan), or a navigator or scout image used to plan the further diagnostic imaging protocols.

Thus, according to an embodiment, the low-resolution image has a different magnetic resonance contrast than the further sets of additional k-space lines, and in particular is acquired in a calibration imaging scan performed before the imaging protocol.

The sets of additional k-space lines are acquired in a central region of k-space, wherein the central region is preferably covered by the low-resolution image. However, each set of additional k-space lines typically comprises fewer k-space lines than would be required to reconstruct an image. For example, a set of k-space lines may comprise 1 to 8, preferably 1-4, k-space lines for each 2D slice of a 2D image dataset. In case the image dataset is a 3D image dataset, a set of k-space lines may comprise 2 to 32, preferably 3-8, k-space lines, most preferred 4 k-space lines. The set of additional k-space lines is acquired repeatedly during the imaging protocol, preferably at - at least approximately - regular intervals. "At least approximately", when used in this application, may mean within $\pm 15\%$, preferably within $\pm 10\%$, most preferred within $\pm 5\%$. The intervals at which the sets of additional k-space lines are acquired is preferably between 100ms and 3000ms, more preferred between 500ms and 1500ms. Thus, steps (b2) and (c) are preferably repeated at these intervals throughout the imaging protocol. Each set of additional k-space lines thereby provides information on the position of the subject at the point in time at which the set was acquired, i.e. one set of pose parameters. A motion trajectory of the subject during the imaging protocol is thereby obtained. The pose parameters may be rigid-body motion parameters, wherein each set of pose parameters comprises e. g. three translational and three rotational parameters, or non-rigid pose parameters. "Additional" means that the k-space lines are acquired in addition to those that are required under the imaging protocol to acquire the MR image, i.e. they are redundant when it comes to image reconstruction. The sets of additional k-space lines are typically always acquired at the same position in k-space at each acquisition, but this is not mandatory. Further, the sets of additional k-space lines may all have the same contrast, but that is not a requirement. It is a great advantage of using a trained machine learning model, that pose parameters can also be extracted from sets of guidance lines having different contrasts, e.g. having been acquired at different TEs and/or at different positions during an echo train.

According to an embodiment, the imaging protocol comprises a plurality of echo trains or "shots" wherein one shot comprises a plurality of magnetic resonance echoes, e.g. spin echoes and/or gradient echoes. During each echo, a k-space line is acquired. In most sequences, an echo train comprises an excitation or preparation pulse, and then all echoes have their own excitation/refocussing pulses. There may be 1 to 1024 echoes, preferably 8 to 256 echoes, in one echo train. In 2D TSE sequences, an echo train may typically contain less than 40, preferably less than 30 echoes. Preferably, at least one set of additional k-space lines is acquired during at least some (e.g. more than 80%) of the echo trains.

According to an embodiment, the method further comprises acquiring a reference set of additional k-space lines within a central region of k-space, preferably at the beginning of the imaging protocol, and wherein the trained machine learning model is applied to a dataset comprising the low-resolution image in k-space notation, the reference set of additional k-space lines and a further set of additional k-space lines. Thereby, the trained machine learning model is able to interpret the pose difference between the two sets of guidance lines. Preferably, the reference set of additional k-space lines is the same for each estimation of pose parameters during the imaging protocol.

By including a reference set of additional k-space lines into the dataset, to which the trained machine learning model is applied, the method becomes more robust. In particular, the trained machine learning model can generate accurate pose parameters, even if the low-resolution image has a different contrast than the guidance lines. The reference set of guidance lines and the further set of guidance lines preferably have matching contrast. However, it has been found that the trained machine learning model can even cope with contrast differences. Surprisingly, it has been found that the reference set of additional k-space lines needs not necessarily have been acquired particularly close in time to the lower resolution image. Nevertheless, preferably the reference set of additional k-space lines is acquired at the beginning of the imaging protocol. The low-resolution magnetic residence image is also preferably acquired before or at the beginning of the imaging protocol.

The pose parameters can be used for prospective and/or retrospective motion correction of the magnetic resonance image dataset, for notifying an operator or for triggering further actions.

According to an embodiment, the pose parameters are used to adapt a field-of-view of the magnetic resonance image dataset during the imaging protocol. In other words, they are used for prospective motion correction. This may be done by comparing the latest set of pose parameters with the previous set, thereby finding the difference, e.g. a translation and/or a rotation, and translating and/or rotating the field-of-view of the imaging protocol accordingly. The new field-of-view is then used in the further acquisition of the imaging protocol, for example for the next shot.

According to an embodiment, the pose parameters are analysed and, if the pose parameters indicate that subject motion has been above a certain threshold, the method comprises a step of triggering one or more of alerting a user supervising during the image acquisition; aborting or restarting the acquisition of the magnetic resonance image dataset; and re-acquiring the magnetic resonance data affected by the subject motion.

This is a feature that is only possible because the trained machine learning model is so fast that the pose parameters can be evaluated while the imaging protocol is being performed. Thereby, real-time feedback can be given to the radiologist, e.g. a warning can be issued. For example, the pose parameters can be further processed in order to predict whether aborting the imaging protocol is advantageous, or in order to trigger a re-acquisition of the most corrupted echo trains. Alternatively or additionally, the pose parameters could be used to display the detected motion trajectory.

According to an embodiment, the imaging protocol comprises acquiring a plurality of echo trains (ETs), each echo train comprising a plurality of echoes, wherein one k-space line is sampled during one echo, and wherein at least one further set of additional k-space lines is acquired in at least some of the echo trains, preferably at the beginning of the echo train, and wherein the pose parameters estimated from a further set of additional k-space lines is used to adapt a field-of-view of the magnetic resonance image dataset in the same echo train in which the further set of additional k-space lines is acquired, or in the next echo train. This has the advantage that very fast prospective motion correction is possible. Since an echo train may have a duration of e.g. between 100ms and 3000ms, more preferred between 500ms and 1500ms, correcting the field-of-view after each echo train already gives a very good motion correction. However, the inventive method is so fast that an adaptation of the field-of-view is possible even during one echo train, e.g. 1 to 20, preferably 1 to 5 times per echo train. Thereby, patient motion can be corrected at a time resolution of for example between 30ms and 200ms, more preferred between 50ms and 150ms.

In other words, the trained machine learning model allows to increase the number of GL sets per ET. For example, a set of GLs can be acquired at the beginning, middle and end of an ET. Alternatively, one GL set can be acquired before every echo. Thereby, the temporal resolution can be increased. In a further embodiment, the GLs can be positioned at any different time point instead of at the standard -TR/2 time point during the sequence run, e.g. during contrast waiting times. In SAMER, for each position of GL during the echo train, a separate scout has to be acquired. This is not required for the method of the invention, provided the network is trained accordingly. In a specific implementation, the NN can use GLs at the beginning of each ET or at any time before the ET for fast detection and prospective correction of the ET, thereby closing k-space gaps and accounting for spin history effects.

According to an embodiment, the magnetic resonance image dataset is acquired using a multi-channel coil array, and wherein the low-resolution image in k-space notation and the further set of additional k-space lines are pre-processed by removing a peripheral region of k-space and/or by reducing a number of channels. Thereby, the dataset which is input into the trained machine learning model is cropped to a smaller size, making the processing faster.

According to an embodiment, the trained machine learning model comprises neural network. According to an embodiment, it comprises a convolutional neural network (CNN), in particular a DenseNet. A DenseNet is a type of convolutional neural network that utilises dense connections between layers, through dense blocks, where all layers having matching feature-map sizes are connected directly with each other. Thereby, a fast NN can be realised. The NN may e.g. utilize 3D complex convolutions with dilation of 8. The NN may comprise 2 to 4 dense blocks, each dense block followed by a transition layer. The transition layer reduces the number of channels and may in the first and second layer also reduce the spatial size, e.g. by 2x2x2.

Other deep learning architectures are also possible. For example, the trained machine learning model may comprise a transformer-based network.

The NN operates exclusively in k-space. The input to the NN preferably is a dataset comprising three components, each in k-space notation:

1. a reference set of guidance lines, which is e.g. the first set of guidance lines acquired during the imaging protocol, for example acquired during the first echo train,
2. the set of GL from which the pose parameters are to be extracted, and
3. a low-resolution image, e.g. a calibration or reference scan.

Before applying the NN, the k-space data may be pre-processed, in particular to reduce the amount of data and thus to make the NN faster. For example, the GLs and the low-resolution image may be coil-compressed, wherein the number of channels is reduced e.g. from 16 to 4. Further, the k-space data may be cropped to cover only a central portion of k-space. Thereby, the dataset may achieve a size of for example 32x16x16x4x3x2, wherein the dimensions are $k_x$ x $k_y$ x $k_z$ x no. of coil channels x no of components (reference GL, GL and low-resolution image) x 2 (complex numbers). The output of the NN is preferably a set of rigid motion pose parameters, i.e. three translational and three rotational parameters.

According to an embodiment, the pose parameters are used for retrospective motion correction of the magnetic resonance image dataset. Thereby, the pose parameters are used in the image reconstruction, which may use a forward model incorporating motion parameters, as described below. Alternatively, a second machine learning model, e.g. a second NN, may be trained to reconstruct the diagnostic image from the k-space data and the motion trajectory obtained from the GLs.

When using the pose parameters for retrospective motion correction, preferably a method for generating a motion-corrected magnetic resonance image dataset of an object is carried out, the method comprising receiving k-space data acquired using the acquisition method according to an embodiment of the invention, receiving estimated pose parameters for each further set of additional k-space lines, and estimating the motion-corrected magnetic resonance image dataset by minimizing the data consistency error between the k-space data acquired in the imaging protocol and a forward model described by an encoding matrix, wherein the encoding matrix includes the pose parameters for each further set of additional k-space lines, Fourier encoding, and optionally subsampling and/or coil sensitivities of a multi-channel coil array. Thereby, retrospective motion correction is possible.

The pose parameters are provided by the trained machine learning model of the inventive method. The motion-corrected image dataset can then be estimated by minimizing the data consistency error between the k-space data acquired in the imaging protocol and a forward model. The forward model is preferably a "SENSE + motion" model, as described in K. P. Pruessmann, M. Weiger, M. B. Scheidegger, and P. Boesiger, "SENSE: sensitivity encoding for fast MRI," Magn. Reson. Med., vol. 42, no. 5, pp. 952-962, 1999.

In an example, the mathematical model used is an extension of SENSE parallel imaging, with rigid-body motion parameters included into the forward model. The forward model or encoding operator $\boldsymbol{E_\theta}$ for a given patient motion vector $\theta$ (comprising pose parameters over time) relates the motion-free image x to the acquired multi-channel k-space data s. At each time point i that is considered, e.g. the acquisition time of the sets of guidance lines, the subject's position is described by a new set of six rigid-body motion parameters $\theta_i$ that describe the 3D position of the object. Accordingly, the multi-channel k-space data $s_i$ acquired at time point $i$ may be related to the 3D image volume x through image rotations $R_i$, image translations $T_i$, coil sensitivity maps C, Fourier operator F and under-sampling mask $M_i$ by the following formula 1

$$s_i = E_{\theta_i} x = M_i\, F\, C\, T_{\theta_i} R_{\theta_i} x \qquad [1]$$

In prior art methods, both the motion corrected image vector $x$ and the motion trajectory $\theta$ are estimated by performing an alternating, repeated optimization between the image vector (formula 2) and the motion vector (formula 3):

$$[\hat{x}] = \operatorname{argmin}_x \left\| E_{\hat{\theta}} x - s \right\|_2 \qquad [2]$$

$$[\hat{\theta}] = \operatorname{argmin}_\theta \left\| E_\theta \hat{x} - s \right\|_2 \qquad [3]$$

This can lead to convergence issues as updates of $x$ and $\theta$ will be computed on inaccurate information. Moreover, the reconstruction is computationally demanding as repeated updates of $x$ (millions of imaging voxels) are needed.

In the method of the invention, the motion trajectory $\theta$ is estimated from the sets of guidance lines and a trained machine learning model, so that equation [3] does not have to be solved. For the final image reconstruction, the pose parameters from each set of guidance lines are combined and the motion-mitigated image is obtained from solving only the standard least-squares problem of equation [2].

According to a further aspect of the invention, a computer-implemented method for providing a trained machine learning model is provided. The method comprises receiving input training data comprising a low-resolution magnetic resonance image of a body part in k-space notation and a set of additional k-space lines from a central region of k-space, wherein the low resolution image has been derived from an example magnetic resonance image, and the set of additional k-space lines has been derived from the same example magnetic resonance image after the image has been rotated and/or translated by a set of pose parameters; receiving output training data comprising the set of pose parameters; training a machine learning model based on the input training data and the output training data; and providing the trained machine learning model. According to an embodiment, the input training data are derived from previously acquired example MR images, for example clinical 3D image datasets, which may be images of the same body part or different body parts from various subjects. These image datasets are optionally modified, e.g. flipped and/or stretched, for optional data augmentation and then shifted and rotated to simulate patient motion, using pre-determined motion trajectories, and Fourier-transformed back into k-space, where the guidance lines are extracted for each pose during the motion trajectory. A low-resolution image is also simulated from the example MR image. Optionally, for data augmentation, the example magnetic resonance image can be derived from a first acquired MR image by image modifications. In an embodiment, such modifications can include one or all of the following steps: image flips, mirroring of the image and stretching. Optionally, real measured motion cases can enter the training, when the respective motion trajectory is determined by a reference method. By augmenting the data, a low number of e.g. 10 to 50 3D acquired MR images may be sufficient to train the machine learning model so that it correctly predicts the pose parameters from the guidance lines and the low-resolution image. According to an embodiment, a set of reference guidance lines is also used as input training data.

According to a further aspect of the invention, a computer program is provided which includes program code, which causes a magnetic resonance imaging apparatus - such as the apparatus described herein - to execute the inventive method, in particular the inventive method for acquiring an MR image dataset. However, the program code may also encode the described training method, and the program code may run on a computer as described herein.

According to a further aspect, the invention is directed to a non-transitory computer-readable medium containing a computer program as described herein. The computer-readable medium may be any digital storage medium, such as a hard disc, a cloud, an optical medium such as a CD or DVD, a memory card such as a compact flash, memory stick, a USB-stick, multimedia stick, secure digital memory card (SD) etc.

In a further aspect of the invention, a magnetic resonance imaging apparatus is provided which comprises a radio frequency controller configured to drive an RF-coil, preferably comprising a multi-channel coil-array, a gradient controller configured to control gradient coils, and a control unit configured to control the radio frequency controller and the gradient controller to execute the imaging protocol according to the invention. The MRI apparatus further comprises a processing unit configured to apply a trained machine learning model to a dataset comprising a low-resolution image in k-space notation and a set of additional k-space lines, wherein a set of pose parameters is generated. The MRI-apparatus may be a commercially available MRI-apparatus which has been programmed to perform the method of the invention. The processing unit may be part of the control unit, or it may be separate. The processing unit can in particular be any CPU or GPU. It may be part of a computer, wherein the computer may be a PC, a server, a console of an MRI apparatus, but

EP 4 624 971 A1

it also may be a computer that is remote from the MRI apparatus, it may be connected with it through the internet. Accordingly, the computer may also be a cloud computer, a remote server etc. The computer may also be a mobile device, such as a laptop, tablet computer or mobile phone. The invention is also directed to such a processing unit, i.e. a processing unit configured to apply a trained machine learning model to a dataset comprising a low-resolution image in k-space notation and a set of additional k-space lines, wherein a set of pose parameters is generated.

All features disclosed with regard to the acquisition method may be combined with all features of the training method and vice versa. Also, all features of the disclosed methods may be embodied in the MRI apparatus, computer program and computer-readable storage medium according to other aspects of the invention and vice versa.

The accompanying drawings illustrate various example embodiments of various aspects of the invention. In the drawings:

Fig. 1    is a schematic representation of an MRI apparatus according to an embodiment of the invention;

Fig. 2    is a schematic representation of a three-dimensional k-space;

Fig. 3    is a flow diagram illustrating a method according to an embodiment of the invention;

Fig. 4    is a schematic diagram illustrating a NN architecture;

Fig. 5    is a schematic timeline of an imaging protocol illustrating embeddings of the GLs for retrospective motion correction according to a first embodiment;

Fig. 6    is a schematic timeline of an imaging protocol illustrating embeddings of the GLs for retrospective motion correction according to a second embodiment;

Fig. 7    is a schematic timeline of an imaging protocol illustrating embeddings of the GLs for prospective motion correction according to a first embodiment;

Fig. 8    is a schematic timeline of an imaging protocol illustrating embeddings of the GLs for prospective motion correction according to a second embodiment;

Fig. 9    is a schematic timeline of an imaging protocol illustrating embeddings of the GLs for prospective and retrospective motion correction according to a first embodiment.

Similar elements are designated with the same reference signs in the drawings.

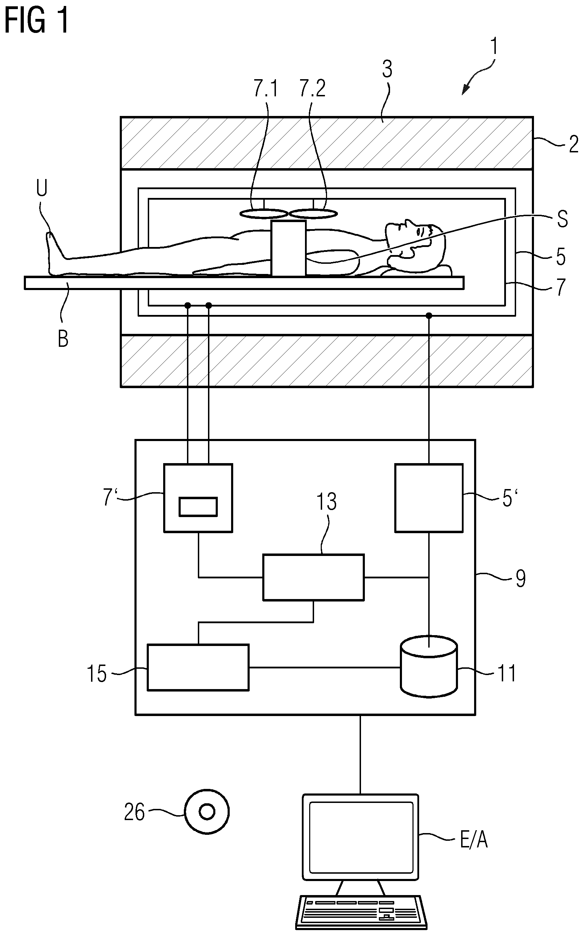

Fig. 1 schematically shows a magnetic resonance (MR) apparatus 1. The MR apparatus 1 has an MR data acquisition scanner 2 with a magnet 3 that generates the constant magnetic field, a gradient coil arrangement 5 that generates the gradient fields, one or several radiofrequency (RF) antennas 7 for radiating and receiving RF signals, and a control computer 9 configured to perform the inventive method. The radio-frequency antennas 7 may include a multi-channel coil array comprising at least two coils, for example the schematically shown coils 7.1 and 7.2, which may be configured to transmit and/or receive RF signals (MR signals).

In order to acquire MR data from a 3D volume S of an examination subject U, for example a patient or a phantom, the subject U is introduced on a bed B into the measurement volume of the scanner 2. MR data can be acquired using a method according to an embodiment of the invention from the volume S. The control computer 9 controls the MR apparatus 1, and the gradient coil arrangement 5 with a gradient controller 5' and the RF antenna 7 with a RF transmit/receive controller 7'. The RF antenna 7 has multiple channels corresponding to the multiple coils 7.1, 7.2 of the coil arrays, in which signals can be transmitted or received. The control computer 9 also has an imaging protocol processor 15 that determines the imaging protocol. A control unit 13 of the control computer 9 is configured to execute all the controls and computation operations required for acquisitions. The control unit may also perform processing steps, e.g. applying the trained machine learning model. Acquired data or data determined in the process can be stored in a memory 11 of the control computer 9. A user can enter control commands and/or view displayed results, for example image data, an input/output interface E/A. A non-transitory data storage medium 26 can be loaded into the control computer 9 and may be encoded with programming instructions (program code) that cause the control computer 9, and the various functional units thereof described above, to implement any or all embodiments of the method according to the invention.

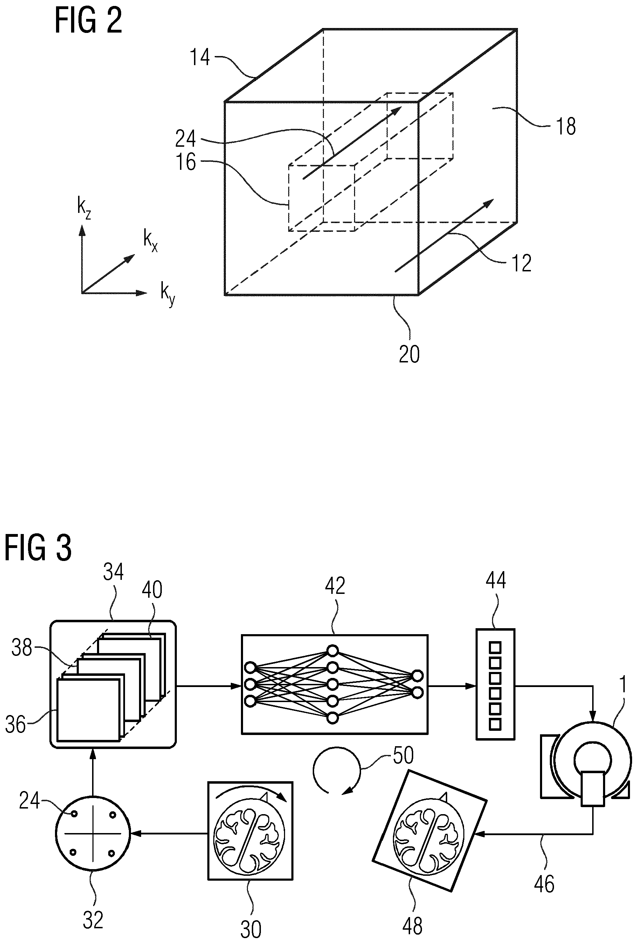

Fig. 2 illustrates a three-dimensional k-space 14 having dimension $k_x$ in frequency encoding (readout) direction, and $k_y$ and $k_z$ in the phase encode plane 20. A k-space line acquired during one echo is illustrated at 12. The k-space volume 14 includes a central region 16 and a periphery 18. The additional k-space lines 24 as well as the k-space lines acquired for the low-resolution scout are located in the central region 16. In the phase encode plane 20, the central region 16 may cover about 1/12 to 1/16 in each direction, i.e. less than 1/100 of the total square phase encode plane 20.

Fig. 3 illustrates a method according to an embodiment of the invention. The illustrated steps are carried out several times during an imaging protocol, during which a diagnostic image is to be acquired, as indicated by arrow 50. Starting at 30, the patient might rotate his head, which is the body part of which an image is taken, during the imaging protocol. Directly after the motion, a set of guidance lines 32 is acquired. The picture at 32 shows the phase encode plane and four guidance lines 24, one in each quadrant. The data from the set of guidance lines 32 is copied several times into each quadrant of a component of the input dataset for the NN, as illustrated at 34. The input dataset 34 to the NN comprises the guidance lines 36 which have just been acquired at 32, a set of reference GLs 38, which have been acquired some time earlier during the imaging protocol, and the low-resolution image dataset 40, for example a calibration scan. The NN illustrated at 42 is applied to the input dataset 34, and the output is a set of pose parameters 44. These pose parameters are used by the MRI apparatus 1 to adjust the field-of-view e.g. by updating the gradient and frequency settings. Because the NN requires so little processing time, this can be done practically in real time after acquiring the GLs 24. Thus, the imaging protocol will continue with an updated field-of-view, as illustrated at 48. This sequence may be carried out once or even several times during one echo time.

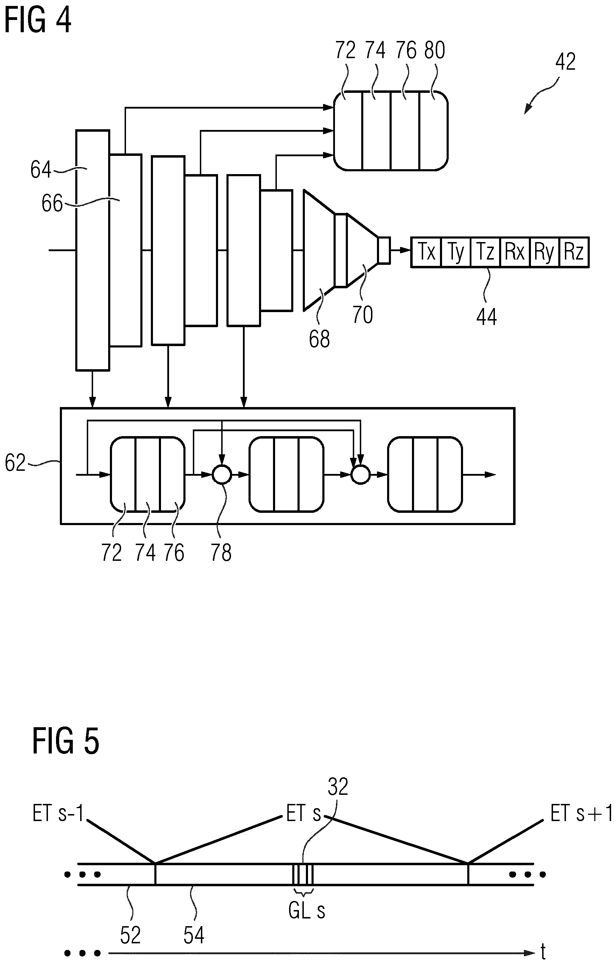

Fig. 4 illustrates an example NN 42 which can be used as trained machine learning model. The NN 42 may comprise 2 to 4 dense blocks 64, each dense block followed by a transition layer 66. The dense blocks 64 may utilize 3D complex convolutions with dilation of 8. The transition layer 66 reduces the number of channels and may in the first and second layer also reduce the spatial size by 2x2x2. A dense block is further illustrated at 62, showing that each dense block has a batch normalisation layer 72, followed by an activation function 74, followed by a complex convolution 76. Concatenation is illustrated at 78. The transition layers 66 comprise a batch normalisation 72, followed by an activation function 74, followed by a complex convolution 76, followed by max. pooling 80. In this example, the first DenseBlock and last transition layer uses tanh activation, the rest uses leaky ReLU. The last block of the NN maps the feature vector to the pose parameters 44 in two linear layers 68, 80 with tanh activation.

Figs. 5 to 9 shows sketches of different exemplary embeddings of the GLs and NN for a retrospective, prospective and combined motion correction. The horizontal bars 52 indicate the timeline of the imaging protocol. The bars 52 are divided into echo trains (ETs) 54. The position of each GL set - in this example, each set comprises 4 additional k-space lines - is indicated by four vertical bars 32. However, the number of lines within one set and the number of GL sets acquired as well as the number of GL sets used for prospective and retrospective correction is flexible and any combination is possible according to the invention. A shading indicates a prospective adaption of the field-of-view to the motion estimate of the NN. A retrospective estimation may also correct only parts of the ET and/or parts of neighbouring ETs.

Fig. 5 illustrates a case where the pose parameters derived from the GLs are used for retrospective motion correction only. The set of GLs 32 is acquired in the middle of an ET 54, and the diagnostic image dataset is reconstructed using the pose parameters and a motion model after the acquisition.

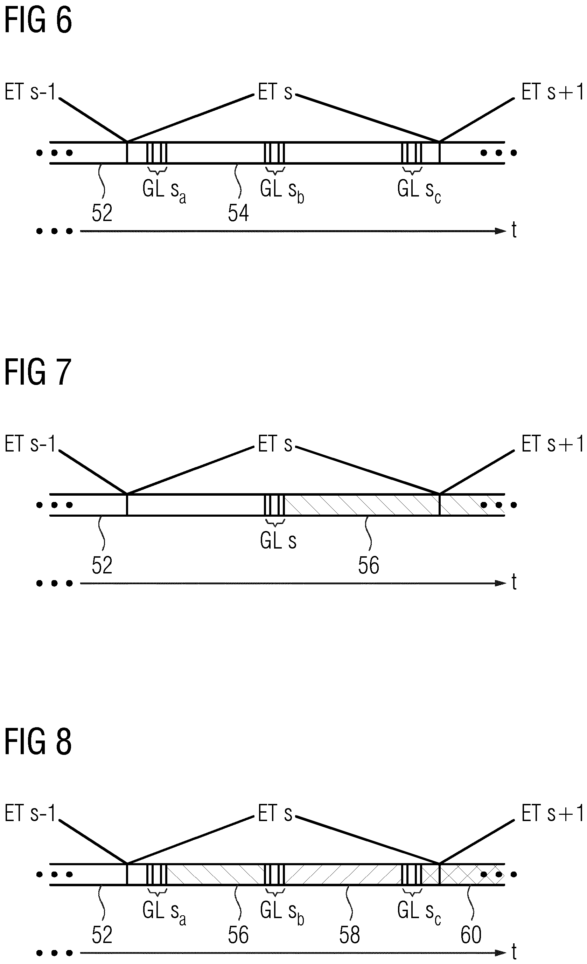

Fig. 6 illustrates retrospective motion correction using multiple - here three - GL sets 32 per echo train 54. Motion parameters are estimated for every GL set 32, and the diagnostic image dataset is reconstructed using the pose parameters and a motion model after the acquisition.

Fig. 7 illustrates prospective motion correction with a single GL set 32 acquired per echo train. After the acquisition of each GL set 32, pose parameters are estimated and the field-of-view is adapted to this pose directly afterwards, in the shaded area 56.

Fig.8 illustrates prospective motion correction with multiple GL sets 32 acquired per echo train. After the acquisition of each GL set 32, pose parameters are estimated and the field-of-view is adapted to this pose in the shaded areas 56, 58, 60.

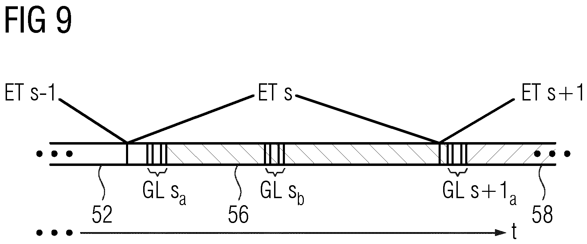

Fig.9 illustrates prospective and retrospective motion correction with multiple GL sets 32 acquired per echo train. GL set $s_a$ is acquired in echo train s, GL set $s+1_a$ is acquired in echo train s+1. After the acquisition of GL sets $s_a$ and $s+1_a$, which are acquired at the beginning of their respective echo trains, pose parameters are estimated and the field-of-view is adapted to this pose for the remaining ET in the shaded areas 56, 58. Residual artefacts can be corrected retrospectively using the GL sets $s_b$ acquired in the middle of each echo train.

## Claims

1. A method for acquiring a magnetic resonance image dataset of a body part of a subject (U),

    wherein the magnetic resonance image dataset is acquired using an imaging protocol, wherein spatial encoding is performed using phase encoding gradients along at least one phase encoding direction ($k_y$, $k_z$), and frequency encoding gradients along a frequency encoding direction ($k_x$), and wherein k-space (14) is sampled during the imaging protocol by acquiring a plurality of imaging k-space lines (12, 24) oriented along the frequency encoding direction, and having different positions in the at least one phase encoding direction ($k_y$, $k_z$),

the method comprising the steps of:

(a) acquiring or providing a low-resolution magnetic resonance image (40) of the body part,
(b1) optionally acquiring a reference set (38) of additional k-space lines within a central region (16) of k-space, preferably at the beginning of the imaging protocol,
(b2) acquiring further sets (36) of additional k-space lines (12) within a central region (16) of k-space at intervals throughout the imaging protocol,
(c) applying a trained machine learning model (42) to a dataset (34) comprising the low-resolution image (40) in k-space notation and a further set of additional k-space lines (36), wherein a set of pose parameters (44) is generated, the pose parameters being an estimation of the pose of the subject during the acquisition time of the further set of additional k-space lines.

2. The method of claim 1, wherein the pose parameters (44) are used for prospective (46) and/or retrospective motion correction of the magnetic resonance image dataset, for notifying an operator and/or for triggering further actions.

3. The method of claim 1 or 2, wherein the method further comprises,

acquiring a reference set (38) of additional k-space lines within a central region (16) of k-space, preferably at the beginning of the imaging protocol,
and wherein the trained machine learning model (42) is applied to a dataset comprising the low-resolution image (40) in k-space notation, the reference set (38) of additional k-space lines and a further set (36) of additional k-space lines.

4. The method of any one of the preceding claims, wherein the pose parameters (44) are used to adapt (46) a field-of-view of the magnetic resonance image dataset during the imaging protocol.

5. The method of any one of the preceding claims, wherein the pose parameters (44) are used for retrospective motion correction of the magnetic resonance image dataset.

6. The method of any one of the preceding claims, wherein the pose parameters (44) are further processed and, if the pose parameters indicate that subject motion has been above a certain threshold, the method comprises a step of triggering one or more of

- alerting a user supervising during the image acquisition;
- aborting or restarting the acquisition of the magnetic resonance image dataset;
- re-acquiring the magnetic resonance data affected by the subject motion.

7. The method of any one of the preceding claims, wherein the low-resolution image (40) has a different magnetic resonance contrast than the further sets (36) of additional k-space lines, and in particular is acquired in a calibration imaging scan performed before the imaging protocol.

8. The method of any one of the preceding claims, wherein the imaging protocol comprises acquiring a plurality of echo trains (54), each echo train comprising a plurality of echoes, wherein one k-space line is sampled during one echo,

and wherein at least one further set of additional k-space lines (32) is acquired in least some of the echo trains (54), preferably at the beginning of the echo train,
and wherein the pose parameters (44) estimated from a further set of additional k-space lines is used to adapt a field-of-view of the magnetic resonance image dataset in the same echo train (54) in which the further set of additional k-space lines is acquired, or in the next echo train.

9. The method of any one of the preceding claims, wherein the magnetic resonance image dataset is acquired using a multi-channel coil array (7.1, 7.2), and wherein the low-resolution image (40) in k-space notation and the further set of additional k-space lines are pre-processed by removing a peripheral region of k-space and/or by reducing a number of channels.

10. The method of any one of the preceding claims, wherein the trained machine learning model (42) comprises a convolutional neural network, in particular a DenseNet.

11. A method for generating a motion-corrected magnetic resonance image dataset of an object, comprising:

receiving k-space data (14) acquired using the acquisition method according to any one of claims 1 to 9;
receiving estimated pose parameters (44, $\theta_i$) for each further set of additional k-space lines (12) obtained using the method according to any one of claims 1 to 9; and
estimating the motion-corrected magnetic resonance image dataset (x) by minimizing the data consistency error between the k-space data (14) acquired in the imaging protocol and a forward model described by an encoding matrix, wherein the encoding matrix includes the pose parameters ($\theta_i$) for each further set of additional k-space lines (12), Fourier encoding (F), and optionally subsampling (M) and/or coil sensitivities (C) of a multi-channel coil array (7.1, 7.2).

12. A computer program including program code, which causes a magnetic resonance imaging apparatus (1) to execute the method according to any one of claims 1 to 11.

13. A computer-implemented method for providing a trained machine learning model, comprising:

- receiving input training data (34) comprising a low-resolution magnetic resonance image of a body part in k-space notation and a set of additional k-space lines from a central region (16) of k-space, wherein the low resolution image has been derived from an example magnetic resonance image, and the set of additional k-space lines has been derived from the same example magnetic resonance image after the image has been rotated and/or translated by a set of pose parameters,
- receiving output training data comprising the set of pose parameters (44);
- training a machine learning model (42) based on the input training data and the output training data; and
- providing the trained machine learning model.

14. A computer program including program code, which causes a computer to execute the method according to claim 13.

15. A magnetic resonance imaging apparatus (1) configured to execute the method of one of claims 1 to 11, comprising:

a radio frequency controller (7') configured to drive an RF-coil (7) comprising a multi-channel coil array (7.1, 7.2),
a gradient controller (5') configured to control gradient coils (5),
a control unit (13) configured to control the radio frequency controller (7') and the gradient controller (5') to execute the imaging protocol,
a processing unit (13) configured to apply a trained machine learning model to a dataset comprising a low-resolution image in k-space notation and a set of additional k-space lines, wherein a set of pose parameters is generated.

FIG 1

## FIG 2

## FIG 3

# FIG 4

# FIG 5

## FIG 6

ET s-1                    ET s                    ET s+1

• • • •    ‖‖    ‖‖    ‖‖    • • •

GL s$_a$    GL s$_b$    GL s$_c$

52    54    t

• • • •

## FIG 7

ET s-1                    ET s                    ET s+1

• • • •    ‖‖    • • •

GL s

52    56    t

• • • •

## FIG 8

ET s-1                    ET s                    ET s+1

• • • •    ‖‖    ‖‖    ‖‖    • • •

GL s$_a$    GL s$_b$    GL s$_c$

52    56    58    60    t

• • • •

FIG 9

Europäisches
Patentamt

European
Patent Office

Office européen
des brevets

# EUROPEAN SEARCH REPORT

**Application Number**

EP 24 16 7445

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | POLAK DANIEL ET AL: "Motion guidance lines for robust data consistency-based retrospective motion correction in 2D and 3D MRI", MAGNETIC RESONANCE IN MEDICINE, vol. 89, no. 5, 6 February 2023 (2023-02-06), pages 1777-1790, XP093047025, US ISSN: 0740-3194, DOI: 10.1002/mrm.29534 | 1-3,5,7, 9-15 | INV. G01R33/54 G01R33/56 G01R33/567 |
| A | * page 1779 - page 1783; figures 1, 2, 5; table 1 * | 4,6,8 | |
| A,D | POLAK DANIEL ET AL: "Scout accelerated motion estimation and reduction (SAMER)", MAGNETIC RESONANCE IN MEDICINE, WILEY-LISS, US, vol. 87, no. 1, 13 August 2021 (2021-08-13), pages 163-178, XP002806334, ISSN: 0740-3194, DOI: 10.1002/MRM.28971 * the whole document * | 1-15 | |
| | | | TECHNICAL FIELDS SEARCHED (IPC) |
| | | | G01R |

The present search report has been drawn up for all claims

| Place of search | Date of completion of the search | Examiner |
|---|---|---|
| Munich | 30 August 2024 | Raguin, Guy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **L. CORDERO-GRANDE ; R. TEIXEIRA ; E. HUGHES ; J. HUTTER ; A. PRICE, HAJNAL**. Sensitivity Encoding for Aligned Multishot Magnetic Resonance Reconstruction. *IEEE Trans. Comput. Imaging*, 2016, vol. 2 (3), 266-280
- **D. POLAK ; D. N. SPLITTHOFF ; B. CLIFFORD ; W.-C. LO ; S. HUANG ; J. CONKLIN ; L. L. WALD ; K. SETSOMPOP ; S. CAULEY**. Scout accelerated motion estimation and reduction (SAMER). *Magn. Reson. Med.*, 2022, vol. 87, 163-178, https://doi.org/10.1002/mrm.28971
- **D. POLAK ; J. HOSSBACH ; D. N. SPLITTHOFF et al.** Motion guidance lines for robust data consistency-based retrospective motion correction in 2D and 3D MRI. *Magn. Reson. Med.*, 2023, vol. 1-14
- **MACLAREN, J. ; HERBST, M. ; SPECK, O. et al.** Prospective motion correction in brain imaging: A review.. *Magn Reson Med*, 2013, vol. 69, 621-636
- **HOSSBACH J ; SPLITTHOFF D ; CAULEY S et al.** Deep Learning-Based Motion Quantification from k-space for Fast Model-Based MRI Motion Correction. *Med. Phys.*, 2023, vol. 50, 2148-2161
- **D. POLAK et al.** *Magn. Reson. Med.*, 2022, vol. 87, 163-178
- *Magn. Reson. Med.*, 2023, 1-14
- **K. P. PRUESSMANN ; M. WEIGER ; M. B. SCHEIDEGGER ; P. BOESIGER**. SENSE: sensitivity encoding for fast MRI. *Magn. Reson. Med.*, 1999, vol. 42 (5), 952-962